Europäisches Patentamt

(19) European Patent Office

Office européen des brevets

(11) Publication number: **0 020 742**
**B1**

(12) **EUROPEAN PATENT SPECIFICATION**

(45) Date of publication of patent specification: **28.09.83**

(51) Int. Cl.³: **C 08 L 33/26, C 08 K 5/05, A 61 F 5/44, A 61 L 15/06**

(21) Application number: **80900175.3**

(22) Date of filing: **17.12.79**

(86) International application number:
**PCT/US79/01109**

(87) International publication number:
**WO 80/01384 10.07.80 Gazette 80/15**

(54) **SHAPED ARTICLES MADE FROM CROSSLINKED ACRYLAMIDE POLYMER COMPOSITIONS.**

(30) Priority: **28.12.78 US 974007**
**13.09.79 US 75021**

(43) Date of publication of application:
**07.01.81 Bulletin 81/1**

(45) Publication of the grant of the patent:
**28.09.83 Bulletin 83/39**

(84) Designated Contracting States:
**AT CH DE FR GB LU NL SE**

(56) References cited:
**GB - A - 1 192 581**
**GB - A - 1 200 106**
**US - A - 3 877 431**
**US - A - 4 051 086**
**US - A - 4 074 039**
**US - A - 4 078 568**
**US - A - 4 153 055**

(73) Proprietor: **C.R. Bard, Inc.**
**731 Central Avenue**
**Murray Hill, New Jersey 07974 (US)**

(72) Inventor: **KRSEK, George**
**P.O. Box 187**
**Culver, IN 46511 (US)**

(74) Representative: **Oliver, Roy Edward et al,**
**POLLAK,MERCER & TENCH High Holborn House**
**52-54 High Holborn**
**London WC1V 6RY (GB)**

Courier Press, Leamington Spa, England

Shaped articles made from crosslinked acrylamide polymer compositions

BACKGROUND OF THE INVENTION

This invention relates to crosslinked acrylamide polymer compositions and shaped polymeric products derived therefrom suitable for application to human skin. More particularly, it is directed to ostomy sealing means (hereinafter called "ostomy seals") made from particular crosslinked compositions having a good balance of physical properties and improved resistance to degradation in service.

The term "Ostomy" has come into use to define, in a broad sense, the surgical procedures known as colostomy, ileostomy, cecostomy, ureterostomy, ileal conduit, ileal bladder, wet colostomy, etc. This surgery usually results in an artifical opening through the abdominal wall for the terminal end of the intestine or a duct, called a stoma, to discharge the body wastes of feces or urine.

Many ostomy devices or appliances have been developed through the years to aid the ostomy patient. Typical of such devices are ostomy bags or pouches constructed of flexible plastics or rubber materials, for receiving and holding these body wastes. In use it is necessary that a liquid-proof seal be maintained between the patient's skin and the bag (or retainer plate to which the bag may be attached) to prevent leakage of the waste material onto the skin of the patient surrounding the stoma or even therebeyond. In addition to the odor that would result form such leakage, irritation of the peristomal skin of the patient surrounding the stoma is extremely likely under circumstances of leakage, and in ileostomy cases, where the discharge is from the small intestine, digestive liquids can actually digest the skin of the patient if they come in contact with it.

Consequently, extensive efforts have been directed to developing sealing materials and means that provide effective liquid-tight seals with minimum irritation to the peristomal skin. Ideally an ostomy seal should be soft and flexible so as to conform to the body, yet have sufficient elasticity and recovery to firmly engage the stoma. It should be non-allergenic non-irritating and non-sensitizing; and desirably possess visual appeal and freedom from odor. Further, it should be resistant to acids, bases, enzymes and other materials which may be found in intestinal and urinary discharges. Lastly, and possibly most critical, it should be capable of absorbing moisture from the skin and from any body wastes it contacts, without disintegrating or developing a slimy surface so as to maintain useful and serviceable cohesive and adhesive properties. Of equal importance, all these properties must be provided with sealing compositions that are both economical and easily fabricated into ostomy seals, which may be in the form of pads, gaskets, rings and the like as is well-known to the art.

SUMMARY OF THE INVENTION

While a number of compositions have been developed for ostomy seals, none satisfy all the prerequisites of an ideal seal. Consequently, it is an object of this invention to provide an improved ostomy seal (and ostomy appliances utilizing such a seal) that more completely and nearly satisfies these requirements. These and still other objects and advantages, which will become apparent from the following description and claims are attained with an ostomy seal that is made with a composition comprising: a water-dispersible acrylamide polymer; a quantity, rendering the composition soft and elastomeric, of a solvating water-miscible polyol, or mixture of polyols, containing water; and a crosslinking polyaldehyde. After the composition is formed into the desired seal configuration, the seal is treated to effect crosslinking of the acrylamide polymer and thereby render it substantially insoluble in water.

DETAILED DESCRIPTION

In the following description and claims, all parts and percentages are by weight.

The acrylamide polymer used in the ostomy seal composition of the invention is a water-dispersible acrylamide or methacrylamide polymer or copolymer capable of forming at 1% total solids a true aqueous solution and/or a stable hydrocolloidal dispersion. It has been found that "nonionic" virtual homopolymers of acrylamide (containing no more than about four percent by weight sodium acrylate) having high molecular weight, such as Reten 420 (Hercules) (Reten is a Registered Trademark), give seals having, after being crosslinked, 350% or less water swelling (as measured by weight gain) after 24 hours water immersion and, hence, constitutes a preferred embodiment. on the other hand, copolymers containing appreciable quantities of anionic groups (such as Reten 421 and 425 containing 10 percent or more of sodium acrylate) or cationic groups (such as Reten 210 and 220 containing 10 percent or more of betamethacryloyloxyethyltrimethyl ammonium methyl sulfate) give seals, after crosslinking, swelling about 2,250% (Reten 421), 3,800% (Reten 425) and 1,050% (Reten 210). Consequently, for minimum water swellability, the total weight of monomer units having anionic and/or cationic functionality in the copolymer or mixture of copolymers utilized in the seals of the invention preferably will not exceed about 5 percent of the total weight of acrylamide and/or methacrylamide polymers employed; and, ideally, will not exceed about 2 percent of the total weight of the polymer or mixture of polymers for lowest water sensitivity. Equally satisfactory are copolymers of a major portion of acrylamide and/or methacrylamide (51—100 percent) and a minor portion (0—49 percent) of a copolymerisable vinyl monomer or monomer mixture, free of ionic groups, in a quantity not significantly diminishing the polymer's water dispersibility. Useful vinyl comonomers include styrene, vinyl acetate, acrylonitrile, methyl vinyl ether, vinyl pyrrolidone, beta-hydroxyl ethyl and propyl acrylates, methyl acrylate, methyl methacrylate, beta-hydroxy ethyl and propyl methacrylates and vinylidene chloride and even include divinyl monomers, such as divinyl benzene, methylenebisacrylamide and N,N-diallyl-acrylamide, if employed in a quantity small enough (e.g., up to 5 weight percent) not to disrupt the water dispersibility of the acrylamide polymer. Finally, while acrylamide and/or methacrylamide polymers and copolymers are preferred, water-dispersible polymers and copolymers of N-substituted acrylamide or methacrylamide, such as N-methyl acrylamide, N-methyl methacrylamide, N-methylol acrylamide, N-methylol methacrylamide and N-isopropyl acrylamide, may also be used. Hence, in the following description and in the claims, it should be understood that the expression "acrylamide polymer" is used in the generic sense to encompass not only the virtual homopolymers of acrylamide or methacrylamide, but also water-dispersible polymers and copolymers of N-substituted acrylamide or methacrylamide, which are capable of being crosslinked with polyaldehydes, as well as mixtures of any of these polymers. In a preferred embodiment of the invention, a mixture of acrylamide polymers is used and at least 50 percent of the mixture is a virtual homopolymer of acrylamide. The molecular weight of the acrylamide polymer should be high enough so as to give an ostomy seal having, after crosslinking, substantially elastomeric-like properties. Typically, useful polymers have given one percent aqueous solutions (or gels) at 25°C having a Brookfield viscosity of 10 mPa.s or more, and a range of particle sizes such that virtually none is retained on a No. 20 U.S. mesh screen (sieve opening 0.84 mm) while 90—100 percent is retained on a No. 100 U.S. mesh screen (sieve opening 0.149 mm) Polymers having smaller particle sizes may be used, but will gel faster and, hence, may require a compensating reduction in the water level of the polyol(s) and/or temperature of gellation. Because of this, they may be preferred when only a small amount of water, e.g., 1 to 2 percent, is used in the solvating polyol(s).

The water-miscible polyol, or mixture of polyols, containing water that is used to solvate and platicize the acrylamide polymer is chosen to provide a seal composition that is soft, flexible and elastomeric and has no tactile surface exudation of polyol after gellation and crosslinking. Because of its excellent solvating properties and low toxicity and absorption into the skin, glycerine is the preferred polyol. It may be used either by itself or combined with other water-miscible polyols — either of the primary (solvating) or secondary (diluting) type. Other suitable solvating polyols include ethylene glycol, diethylene glycol, and sorbitol (when it is used with sufficient water and/or other polyols to provide a liquid mixture). Useful secondary water-miscible polyols include, without limitation, propylene glycol, dipropylene glycol, the butylene glycols, and polyethylene glycols (above diethylene glycol) having a molecular weight of up to 600. Typically, the plasticizer mixture will comprise 20—99 percent primary polyol, 0—79 percent secondary polyol and 1—20 percent water. The preferred level of water will vary depending on the type of primary polyol and percentage of secondary polyol used. For example, ethylene glycol and/or glycerine, alone, give excellent results with as little as 1 percent water, although 3—7 percent is typically used to accelerate gellation. Diethylene glycol, on the other hand, requires more water, typically 5—15 percent, for good solvating properties. Higher quantities of secondary polyol, as for example 20 percent or more, also necessitate the use of higher water levels, such as 5—15 percent if suitably rapid gellation is to be attained. Additionally, temperature affects the minimum level of water required: more being required when lower gelling temperatures are used. Finally, acrylamide polymers of higher molecular weight and/or less hydrophilic nature may require more water in the polyol plasticizer. In work to date, the best balance of seal properties coupled with good processing and gelling characteristics has been obtained with polyol plasticizer mixtures comprising 50—99 percent glycerine, 0—49 percent secondary polyol, and 1—18 percent water. In the above discussion, the percentage of water includes, of course, not only water added to the polyol, but also that present in the polyol as purchased.

The quantity of polyol, or mixtures of polyols, containing water used is sufficient to provide a seal which is, as previously stated, soft and flexible and yet elastomeric. Illustrative of physical properties of seals so obtained are: Shore A, 1—5 (as measured on a Shore Durometer according to 1981 ASTM—35, Designation D2240—8—1) (or Sponge Rubber Gauge values, 80—95); tensile strength at break, 1.10—1.52 bar (16—22 psi); tensile modulus, 0.83—2.07 bar (12—30 psi); elongation at break 350—600 percent; crescent tear, 35.0 bar (507 psi); and compression modulus 1.03—2.41 bar (15—35 psi). Typically, 125 to 200 parts of polyol(s) containing water per 100 parts of acrylamide polymer provides such properties, with 150—175 parts being the generally preferred range. If the seal composition contains appreciable quantities of particulate fillers, such as clay, calcium carbonate, calcium silicate or silicon dioxide, or other types of water-dispersible polymers, as hereinafter described, quantities of polyol(s) in excess of 200 parts may be used or even be required to provide the desired softness and flexibility.

The ostomy seal of the invention is crosslinked with a polyaldehyde which is soluble, in the quantity employed, in the polyol(s)-water mixture used to plasticize the acrylamide polymer. Suitable polyaldehydes include, without limitation, glyoxal, succinaldehyde, glutaraldehyde, 3-methyl glutaraldehyde and alphahydroxyadipaldehyde. Alternatively aldehyde bisulfite addition products, either preformed (e.g. glutaraldehyde bissodium bisulfite) or formed in situ in the polyol-water mixture at the time of formulating and admixing the seal composition, may be used. Such complexes are particularly preferred for providing about one-half or more of the polyaldehyde when diminished yellowing of the seal is desired. While as little as 0.1 part of polyaldehyde per 100 parts of the acrylamide polymer will usually provide acceptable crosslinking, 0.2—0.5 parts are typically employed to ensure adequate crosslinking, and hence acceptable decreased water sensitivity. While more than 0.5 parts may be used, the small additional crosslinking obtained generally does not justify the added cost. The type and quantity of polyaldehyde employed should, as pointed out above, be soluble in the polyol-water plasticizer mixture for maximum crosslinking efficiency. Further, mixtures of suitable polyaldehydes and/or aldehyde bisulfite addition products may be used.

When, as described above, it is desired to form the aldehyde bisulfite addition product in situ, sodium metabisulfite, or sodium bisulfite, and the polyaldehyde are admixed and dissolved in the polyol(s)-water plasticizer mixture prior to admixing the acrylamide polymer. To facilitate solvating and mixing, the bisulfite may first be predissolved in some or all of the water. Sodium bisulfite or the aldehyde bisulfite addition product provides two advantages. First, it lowers the vapor pressure and hence odor of the polyaldehyde during the mixing, fabrication and curing of the seal composition. Secondly, it acts as a color stabilizer to provide a seal composition having less tendency to yellow during the crosslinking treatment and/or in long-term storage, thus giving a seal having less discoloration. For maximum benefit, 1.2 to 2 moles of the metabisulfite salt (or 2.4 to 4 moles of the bisulfite salt) are employed for every two aldehyde equivalents present in the polyaldehyde. Thus to obtain maximum whiteness with a dialdehyde crosslinker, 1.2 to 2 moles of sodium metabisulfite, or 2.4 to 4 moles of sodium bisulfite, would be used per mole of the dialdehyde.

Lesser quantities of the bisulfite may be used, such as 0.5 to 1.2 moles of bisulfite per mole of aldehyde group, when some yellowing is acceptable. As is apparent, some or all of the bisulfite (depending upon the ratio desired) may be supplied by a preformed aldehyde-bisulfite addition product of the polyaldehyde. When only color stabilization is desired, other antioxidants soluble, in the quantity employed, in the polyol-water mixture, such as ascorbic acid, hydroquinone and sodium thiosulfate, may be used to replace, or sometimes supplement, the bisulfite salt as the color stabilizer.

In some instances, the seal composition is enhanced by including formaldehyde, either aqueous (e.g. 37 percent) or solid (e.g. paraformaldehyde) in an amount providing from 0.01 to 1.0 parts of formaldehyde per 100 parts of the acrylamide polymer, with 0.05 to 0.5 parts being typical. At higher levels (e.g., 0.05 parts and more) the seal is aseptic, seal adhesion to the ostomy bag (or bag retainer) is improved, the seal composition appears less grainy and more homogeneous, and the seal swells less in water.

The ostomy seal is typically made by blending the particulate acrylamide polymer or polymers and any solid adjuvant (pigment, colorant, antiseptic, stabilizer and the like) being used, and admixing this blend with the polyol, or mixture of polyols, containing water to form a homogeneous admixture. Typically, the polyaldehyde, and the bisulfite if used, are dissolved in the polyol(s)-water mixture before the acrylamide polymer. Other adjuvants, soluble in the glycols and/or water, likewise may be predispersed in the glycol(s)-water mixture. Sometimes, it may be advantageous to prewet and predisperse the polymer and/or solid adjuvants with some or all of the polyol(s) before admixing the water. These and still other techniques of combining and mixing the seal ingredients will be apparent to those skilled in the art.

After all the ingredients are well dispersed, the resulting liquid dispersion is formed into the desired seal configuration, such as ring, pad, gasket and the like, by casting the composition into an appropriate mold and then gelling and crosslinking the composition. If desired, two or more layers of different compositions may be successively cast, or be combined after gelling, to provide a seal having different properties for each face. The time required for gellation will vary depending upon the seal composition — particularly the water level — and temperature. Generally, one or both are set — the rate of gellation increasing as the water level and/or temperature increase — to effect incipient gellation within 5—60 minutes. Typically, gelling and crosslinking are done in one step by treating the cast liquid seal at an elevated temperature, such as 45—90°C, for a period sufficient to substantially crosslink and water insolubilize the acrylamide polymer. Because low temperatures can require treatment times as long as 48 hours, generally temperatures of about 60°C or higher are preferred: adequate crosslinking being obtained in one hour @ 60°C or 30 minutes @ 90°C. Also, these higher crosslinking temperatures are required when formaldehyde is used, if its advantages are to be realized. Preferably, the kind and quantity of polyaldehyde (and quantity of formaldehyde if used), and the time and temperature of crosslinking are chosen to provide an ostomy seal increasing no more than 400 percent in weight after 24 hours immersion in water @ 25°C and more and preferably no more than 300 percent. If necessary, excessive water loss from the seal during crosslinking may be prevented by means such as treating the seal in a high humidity environment or enclosing the seal in a moisture barrier.

Other ingredients may be incorporated into the seals of the invention, care being taken to choose adjuvants and quantities that are compatible with the seal composition and do not appreciably diminish its desirable physical and chemical properties or significantly inhibit its crosslinking. Thus, the type and quantity of any adjuvant(s) utilized should not increase the seal water swellability to more than the 400 percent, preferably 300 percent, maximum discussed above. Examples of other ingredients that may be used are: other types of water-soluble polymers both natural and synthetic, such as cornstarch, gelatin, casein, guar gum, carboxy methyl cellulose, high-molecular-weight polyethylene oxide, polyvinyl alcohol, vinyl acetate-maleic halfamide copolymers; antiseptic agents; bactericides; fungicides; polyvalent metal (Ca, Mg, etc.) hydroxides and salts; other types of crosslinking agents, such as methylene bisacrylamide; pigments; dyes; fillers; pH-buffers; tackifiers; deodorants, and the like.

When the seal composition is deficient in tackiness or adhesiveness to the skin and/or the ostomy bag, a suitable adhesive may be applied to one or both faces of the seal by means well known in the art. For example, the surface of the seal, after being gelled and either before or after being crosslinked, may be coated with a suitable liquid adhesive, which is then dried or polymerized to the solid state. Alternatively, the adhesive layer may be performed on a release sheet, and the ostomy seal either cast (before gellation), or laminated (after gellation, and either before or after being crosslinked) to the adhesive layer. Illustrative of adhesives that have been used are Swift No. 45508 (Regd. Trademark) polyvinyl acetate/polyacrylate adhesive, Dow Corning No. 355 (Regd. Trademark) medical-grade adhesive, and 3 M ST-1524 (Regd. Trademark) transfer tape.

EXAMPLES 1—9

Nine ostomy rings (about 16.35 mm ($\frac{1}{4}$ inch) thick) were made with Reten 420 (virtual acrylamide homopolymer containing not more than four percent sodium acrylate and having a Brookfield viscosity at one percent solids in water at 25°C of about 300 mPa.s or more) plasticized with a glycerine/propylene glycol mixture containing 5.6 to 8.7 percent water, and crosslinked with glutaraldehyde (examples 1, 2 and 4 to 8) or alpha-hydroxyadipaldehyde (example 3). Example 4, additionally contained paraformaldehyde; example 8, magnesium hydroxide; and examples 6 and 7, sodium metabisulfite in quantities providing mole ratios of 0.5 to 1.0, respectively, of bisulfite to dialdehyde. Example 9, illustrating prior art ostomy seals, contained no dialdehyde.

The dialdehydes and sodium metabisulfite each were first dissolved in part of the water before being admixed with a mixture of the glycerine and propyiene glycol containing the balance of the water. Within a few minutes, the Reten 420 (and magnesium hydroxide in example 8) was added, and mixed (about two minutes) until the polymer particles were wetted by and homogeneously dispersed in the plasticizer mixture. The resulting liquid dispersion was cast into ostomy ring molds 60 mm O.D., 35 mm I.D. and 6.35 mm deep (2—3/8" O.D., 1—3/8" I.D. and $\frac{1}{4}$" deep) and gelled and crosslinked in a hot air oven (except example 9) at the times and temperatures indicated in the Table. After being crosslinked, two-gram sections of the rings were immersed in water at ambient temperature (about 25°C) and the increase in weight and gel strength measured after varying immersion periods. Further, the color of the rings for Examples 5 to 8, immediately after crosslinking, was observed and recorded.

From the data in the Table, it can be seen from examples 1 to 3 that as little as 0.08 parts of glutaraldehyde crosslinks the acrylamide polymer, but that tighter crosslinking and greater water resistivity are obtained at higher dialdehyde levels; and that alpha-hydroxyadipaldehyde is equally effective. Example 4 demonstrates that even better water resistance provided by the concurrent use of formaldehyde with dialdehyde. Examples 6 and 7 illustrate the color stabilization provided by sodium metabisulfite and the enhanced stabilization that is obtained when the ratio of bisulfite is increased from one-half to one mole per mole of the crosslinking dialdehyde. Even better results were obtained when the bisulfite was increased to a mole ratio of 1.8 : 1 such ostomy seals showing no discernable yellowing even after being aged for six hours at 75°C in a hot air oven. Finally, example 8 illustrates that a filler, such as magnesium hydroxide, may be used in the seals of the invention, with advantage as they provide a great increase in the level of plasticizing polyol(s) that may be used, and a diminution of water swelling.

While the invention has been described and illustrated with reference to certain preferred embodiments thereof, e.g., improved acrylamide polymer compositions and sealing means for ostomy appliances, those skilled in the art will appreciate that various changes, modifications and substitutions can be made therein without departing from the invention detailed herein. For instance, the properties possessed by the polymer compositions of the present invention render such compositions and articles shaped therefrom suitable for use in a variety of surgical or medical applications, i.e., pads, wound coverings, bandages, dressings, self-adherent wraps, adhesives for device securement and the like in accordance with the objects and practices of the present invention.

TABLE

| EXAMPLES | 1 | 2 | 3 | 4 | 5 | 6 | 7 | 8 | 9 |
|---|---|---|---|---|---|---|---|---|---|
| Reten 420 | 100 | 100 | 100 | 100 | 100 | 100 | 100 | 100 | 100 |
| glycerine (anhydrous) | 144 | 144 | 144 | 143.7 | 147 | 147 | 147 | 221 | 144 |
| propylene glycol | 16.7 | 16.7 | 16.7 | 16.6 | 16.7 | 16.7 | 16.7 | 25 | 16.6 |
| water | 9.6 | 10.6 | 15.3 | 9.0 | 10.8 | 10.8 | 10.8 | 16.2 | 11.3 |
| glutaraldehyde[1] | 0.08 | 0.41 | — | 0.44 | 0.41 | 0.41 | 0.41 | 0.62 | — |
| alpha-hydroxyadipaldehyde[1] | — | — | 0.82 | — | — | — | — | — | — |
| paraformaldehyde | — | — | — | 0.17 | — | — | — | — | — |
| sodium metabisulfite[1] | — | — | — | 0.76 | — | 0.40 | 0.80 | — | — |
| magnesium hydroxide | — | — | — | — | — | — | — | 50 | — |
| Crosslinking treatment | 40 minutes @ 70°C | | | 60 minutes @ 70°C | 30 minutes @ 90°C | | | | 11 days @ 25°C[2] |
| Color after crosslinking | — | — | — | — | light yellow | off white | white | yellow | — |
| Weight gain (in percent) after immersion in water (25°C) for: | | | | | | | | | |
| 8 hours | 245 | 210 | — | 195 | — | — | — | — | 250 |
| 10 hours | — | — | — | — | 250 | 250 | 260 | 200 | — |
| 24 hours | — | — | 225 | 255 | 330 | 350 | 360 | 280 | N.M.[3] |
| 72 hours | 560 | 390 | 400[4] | 300 | 415 | 450 | 465 | 355 | — |

0 020 742

| EXAMPLES | 1 | 2 | 3 | 4 | 5 | 6 | 7 | 8 | 9 |
|---|---|---|---|---|---|---|---|---|---|
| Gel strength[5] after immersion in water (25°C) for: | | | | | | | | | |
| 10 hours | | | | | + | + | + | + | $-^6$ |
| 24 hours | | | | + | + | + | + | + | |
| 72 hours | | | | + | + | + | − | + | |

[1]Glutaraldehyde and alpha-hydroxyadipaldehyde were used as 25% aqueous solutions, while sodium metabisulfite was used as a 35% aqueous solution. The solutions were admixed with glycerine propylene glycol/water mixture before the Reten 420.

[2]Ring was not treated at an elevated temperature because of the absence of a dialdehyde crosslinker. After 11 days at 25°C, test rings were completely gelled.

[3]N.M. = Not measurable: sample swelled so much that it could not be handled and weighed.

[4]Weight gain after 132 hours water immersion.

[5]Gel strength measured by centering a 750 gram, 1.9 cm diameter bar upon the center of the water-swollen ring section. A strong ring supports the bar for 30 seconds (denoted by ''+''), while a weak ring does not (denoted by ''−''). A blank space indicates that the test was not run.

[6]Gel strength after eight hours water immersion.

## Claims

1. A shaped polymeric product suitable for application to human skin made from a composition which comprises a water-dispersible acrylamide polymer; a quantity, rendering the composition soft and elastomeric, of a solvating water-miscible polyol, or mixture of polyols, containing water; and a polyaldehyde capable of crosslinking the acrylamide polymer; said polymeric product being treated at a temperature and for a time sufficient to substantially crosslink and water insolubilize the acrylamide polymer.

2. The shaped polymeric product of claim 1 in which the composition further includes formaldehyde.

3. The shaped polymeric product of claim 1 wherein the polyol comprises at least 50 percent glycerine and one percent water.

4. The shaped polymeric product of claim 3 wherein the acrylamide polymer is a copolymer containing at least 51 percent acrylamide.

5. The shaped polymeric product of claim 3 wherein a mixture of acrylamide polymers is used, and at least 50 percent of the mixture is a virtual homopolymer of acrylamide.

6. The shaped polymeric product of claim 3 wherein the acrylamide polymer is a virtual homopolymer of acrylamide.

7. The shaped polymeric product of any preceding claim, in which the composition further includes a stabilizer attenuating product discolouration.

8. An ostomy drainage device, having a seal comprising a shaped polymeric product according to claim 7.

9. An ostomy drainage device, having a seal comprising a shaped polymeric product according to claim 1, 2, 3, 4, 5 or 6.

10. An adherent pad comprising a shaped polymeric product according to any of claims 1 to 7.

## Revendications

1. Produit polymère façonné approprié à l'application à la peau humaine fait d'une composition qui comprend un polymère d'acrylamide dispersible dans l'eau, une quantité, rendant la composition souple et élastomère, d'un polyol ou d'un mélange de polyols solvatants miscibles à l'eau contenant de l'eau, et un polyaldéhyde capable de réticuler le polymère d'acrylamide, ledit produit polymère étant traité à une température et pendant une période suffisantes pour fortement réticuler et insolubiliser dans l'eau le polymère d'acrylamide.

2. Produit polymère façonné selon la revendication 1, dans lequel la composition comprend en outre du formaldéhyde.

3. Produit polymère façonné selon la revendication 1, dans lequel le polyol comprend au moins 50% de glycérine et 1% d'eau.

4. Produit polymère façonné selon la revendication 3, dans lequel le polymère d'acrylamide est un copolymère contenant au moins 51% d'acrylamide.

5. Produit polymère façonné selon la revendication 3, dans lequel on utilise un mélange de polymères d'acrylamide et au moins 50% du mélange est un homopolymère virtuel d'acrylamide.

6. Produit polymère façonné selon la revendication 3, dans lequel le polymère d'acrylamide est un homopolymère virtuel d'acrylamide.

7. Produit polymère façonné selon l'une quelconque des revendications précédentes, dans lequel la composition comprend en outre un stabilisant atténuant l'altération de la couleur du produit.

8. Dispositif d'évacuation pour ostomie ayant un joint comprenant un produit polymère façonné selon la revendication 7.

9. Dispositif d'évacuation pour ostomie ayant un joint comprenant un produit polymère façonné selon l'une quelconque des revendications 1, 2, 3, 4, 5 ou 6.

10. Tampon adhérent comprenant un produit polymère façonné selon l'une quelconque des revendications 1 à 7.

## Patentansprüche

1. Ein geformtes polymeres Produkt, geeignet für die Anwendung auf der menschlichen Haut, hergestellt aus einer Zusammensetzung, umfassend ein in Wasser dispergierbares Acrylamid-Polymer; ein solfatisierendes mit Wasser mischbares Polyol oder Mischungen von Wasser enthaltenden Polyolen in einer Menge, um die Zusammensetzung weich und elastomer zu machen; und einen Polyaldehyd, der das Acrylamid polymer vernetzen kann; wobei das polymere Produkt bei einer Temperatur und über einen Zeitraum behandelt wird, die hinreichen, um das Acrylamid-Polymer im wesentlichen zu vernetzen und wasserunlöslich zu machen.

2. Das geformte polymere Produkt nach Anspruch 1, wobei die Zusammensetzung weiterhin Formaldehyd enthält.

3. Das geformte polymere Produkt nach Anspruch 1, worin das Polyol mindestens 50% Glyzerin und 1% Wasser enthält.

4. Das geformte polymere Produkt nach Anspruch 3, worin das Acrylamid-Polymer ein Kopolymer ist, das mindestens 51% Acrylamid enthält.

5. Das geformte polymere Produkt nach Anspruch 3, worin eine Mischung von Acrylamid-Polymeren verwendet wird und mindestens 50% der Mischung ein virtuelles Acrylamid-Homopolymer ist.

6. Das geformte polymere Produkt nach Anspruch 3, worin das Acrylamid-Polymer ein virtuelles Acrylamid-Homopolymer ist.

7. Das geformte polymere Produkt nach einem oder mehreren der vorhergehenden Ansprüche, worin die Zusammensetzung weiterhin ein Stabilisierungsmittel enthält, das die Produktverfärbung vermindert.

8. Eine Ostomiedrainagevorrichtung mit einem Verschluß, enthaltend geformtes polymeres Produkt nach Anspruch 7.

9. Eine Ostomiedrainagevorrichtung mit einem Verschluß, enthaltend ein geformtes polymeres Produkt nach den Ansprüchen 1, 2, 3, 4, 5, oder 6.

10. Ein selbstklebendes Kissen, enthaltend ein geformtes polymeres Produkt nach einem oder mehreren der Ansprüche 1—7.